# FASCICULE DE BREVET EUROPEEN

(11) **EP 0 350 377 B1**
(45) Date de publication et mention de la délivrance du brevet: **27.04.1994**
(21) Numéro de dépôt: 89401881.1
(22) Date de dépôt: 30.06.1989
(51) Int. Cl.: C09K 5/06

(54) **Emballage réfrigérant comprenant un support réfrigérant à base de particules de polymères absorbant les liquides**
Kühlverpackung mit einem Gefrierträger auf der Basis von flüssigkeitabsorbierenden Polymerpartikeln
Refrigerated parcel comprising a refrigerating support composed of liquid-absorbing polymer particles

(30) Priorité: 02.07.1988 JP 88115/88; 12.06.1989 JP 68249/89 U
(43) Date de publication de la demande: 10.01.1990
(73) Titulaire: UNI-CHARM CORPORATION, Kawanoe-shi Ehime-ken (JP)
(72) Inventeur: Hirayama, Miyuki 407 of Royal Plaza, Katsushika-ku Tokyo (JP); Umemura, Eiji 1-E of Residence Yasuha, Kakegawa-shi Shizuoka-ken (JP); Fujiwara, Masatoshi 1-111 of Uni-Haitsu, Kakegawa-shi Shizuoka-ken (JP); Inagaki, Hiroyuki 6-621 of Uni-Haitsu, Kakegawa-shi Shizuoka-ken (JP)
(74) Mandataire: Cabinet HERRBURGER

(56) Documents cités:
- US-A- 3 559 416
- PATENT ABSTRACTS OF JAPAN, vol. 9, no. 59 (C-270)[1782], 15 mars 1985; & JP-A-59 196 386
- WORLD PATENT INDEX LATEST, Derwent File Supplier, Derwent Publications Ltd, London, GB; & JP-A-62 240 377

## Description

### OBJET DE L'INVENTION

La présente invention concerne un emballage réfrigérant et, plus particulièrement, un emballage réfrigérant comprenant un support de réfrigérant obtenu en dispersant et en fixant des particules de polymères absorbant les liquides, sur un substrat absorbant les liquides, pour maintenir ces particules dispersées sur le substrat, et un emballage destiné à enfermer le support de réfrigérant.

### ART ANTERIEUR

Il est bien connu, par exemple d'après la Gazette de Description Japonaise n^{o} 1987-267 386 et 1987-240 377, d'utiliser comme support de réfrigérant des polymères absorbant l'eau, pour obtenir un effet de refroidissement pendant une longue durée et pour éviter l'exsudation ou le suintement du polymère en fixant les particules du polymère absorbant sur un substrat convenable. Un tel support de réfrigérant selon l'art antérieur pourrait autrement être susceptible d'une telle exsudation ou dispersion, et pourrait également faire apparaître une adhérence de collage en cours d'utilisation et pendant la décongélation du réfrigérant. Par suite, un tel support de réfrigérant a généralement été utilisé en pratique avec un sac associé destiné à enfermer le support de réfrigérant pour améliorer la stabilité de forme et la facilité de manipulation de celui-ci.

Avec les articles de cette nature, une quantité d'eau convenable est introduite dans le sac de manière à être retenue par les particules de polymère absorbant, et à être ensuite gelée avant l'utilisation. Les moyens d'introduction de l'eau dans le sac peuvent être grossièrement classés en deux types à savoir un dispositif de type à soupape destiné à introduire l'eau dans le sac scellé par l'intermédiaire d'une soupape telle qu'un clapet de retenue, et un dispositif de type à plongement destiné à introduire l'eau dans le sac par un certain nombre de perforations ou fentes très fines formées dans le sac, en immergeant le sac dans l'eau.

On a constaté que le dispositif d'introduction d'eau de type à soupape était désavantageux non seulement du fait que l'utilisation de la soupape augmentait corrélativement les coûts de fabrication, mais encore qu'il était impossible d'introduire l'eau dans plusieurs sacs simultanément, c'est-à-dire que l'eau devait être introduite dans chaque sac un par un. Le dispositif de type à plongement permet d'introduire l'eau dans plusieurs sacs simultanément, mais il n'est pas pratique du fait qu'un temps relativement long est nécessaire pour introduire l'eau car les dimensions des perforations ou fentes très fines sont strictement limitées pour éviter les fuites de particules de polymère absorbant risquant de se produire avant et après l'introduction dans l'eau, et également du fait que les particules de polymère absorbant tendent à s'agglomérer en provoquant une introduction irrégulière de l'eau dans le sac.

Il est également à noter que l'on a déjà proposé, par le document "Patent Abstracts of Japan, vol.9, n^{o} 59 (C270)[1782], 15 mars 1985" et le document JP-A-59 196 386, un dispositif de refroidissement constitué par une couche interne absorbante entourée par une enveloppe en un matériau relativement plus hydrophobe, mais présentant néanmoins une certaine perméabilité aux liquides.

Un tel dispositif ne s'est toutefois pas avéré non plus totalement satisfaisant.

### RESUME DE L'INVENTION

La présente demande a pour objet de proposer un emballage réfrigérant capable d'éviter l'exsudation des particules de polymère d'absorption du liquide avant et après l'introduction de ce liquide, d'introduire le liquide dans l'emballage réfrigérant en une courte période de temps, de produire une introduction essentiellement uniforme du liquide dans l'emballage réfrigérant, et par conséquent d'obtenir des effets de refroidissement très avantageux.

Le but de la présente invention est de créer des emballages réfrigérants dont les enveloppes ne collent pas les unes aux autres même si l'on empile plusieurs enveloppes les unes sur les autres, les parties de couches perméables au liquide des enveloppes contenant une certaine quantité d'eau résiduelle qui peut geler.

Le but indiqué ci-dessus est atteint, selon la présente invention, grâce à un emballage réfrigérant destiné à être congelé dans un appareil de refroidissement et comportant en combinaison,
- d'une part
   un support de réfrigérant se présentant sous la forme d'une feuille ou d'une natte qui est composé de :
   a - un substrat absorbant le liquide réalisé sous la forme d'une masse de matériau fibreux et,
   b - des particules de polymère absorbant le liquide se présentant sous la forme de particules sensiblement sphériques dispersées et fixées sur le substrat en enfermant partiellement les fibres de celui-ci, ces particules étant capables d'absorber une quantité d'eau représentant plusieurs dizaines de fois à plusieurs centaines de fois leur propre poids, et
- d'autre part
   une enveloppe en forme de sac de boîte ou analogue constituée à sa partie externe par une paire de couches opposées l'une à l'autre et scellées au niveau de leurs pourtours extérieurs pour fermer l'enveloppe en enfermant le support réfrigérant.

Selon l'invention, cet emballage est caractérisé en ce qu'au moins l'une des couches d'une paire est réalisée en un matériau perméable aux liquides de manière à définir une partie de couches perméable aux liquides permettant l'introduction de liquides à l'intérieur de l'enveloppe et en ce que l'enveloppe comporte une partie de couches imperméable aux liquides, les parties de couches perméables aux liquides et imperméables aux liquides respectives d'enveloppes d'emballages réfrigérants empilés dans un appareil de refroidissement coopérant de façon à éviter tout collage de ces emballages.

Selon une autre caractéristique de l'invention, l'une des couches d'une paire est réalisée en un matériau imperméable aux liquides et définit au moins partiellement la partie de couches imperméable aux liquides.

Selon une autre caractéristique de l'invention l'enveloppe comporte au moins une couche réalisée en un matériau perméable aux liquides et coopérant au moins à sa partie centrale avec une feuille imperméable aux liquides interposée entre cette couche et le support de réfrigérant et définissant au moins partiellement la partie de couches imperméable aux liquides.

Selon une autre caractéristique de l'invention, la portion périphérique de la couche indépendante de la feuille imperméable aux liquides définit au moins partiellement la partie de couches perméable aux liquides.

Selon une autre caractéristique de l'invention, le matériau perméable aux liquides est un tissu fibreux non tissé.

Selon une autre caractéristique de l'invention, le matériau imperméable aux liquides est choisi dans le groupe formé par les matières plastiques et les métaux tels que l'aluminium.

L'emballage réfrigérant ainsi réalisé selon la présente invention, peut être utilisé, après qu'une quantité d'eau convenable ait été introduite dans l'enveloppe pour être absorbée et retenue par le support de réfrigérant, cette eau étant ensuite soumise à un processus de congélation, par exemple pour maintenir l'état de fraîcheur de denrées périssables, sans sortir le support de réfrigérant de l'enveloppe.

### BREVET DESCRIPTION DES DESSINS

- la figure 1 est une vue en plan d'une forme de réalisation de l'emballage réfrigérant présentant l'inconvénient auquel on cherche à remédier conformément à l'invention,
- la figure 2 est une vue en coupe suivant la ligne 2-2 de la figure 1,
- la figure 3 est une vue analogue à celle de la figure 2, représentant une autre réalisation de l'emballage réfrigérant selon la présente invention ;
- la figure 4 est une vue en plan représentant une autre forme encore de réalisation de l'emballage réfrigérant selon la présente invention,
- la figure 5 est une vue en coupe suivant la ligne 5-5 de la figure 4,
- la figure 6 est une vue en coupe suivant la ligne 6-6 de la figure 4 ;
- la figure 7 est une vue en plan représentant une autre forme encore de réalisation de l'emballage réfrigérant selon la présente invention ;
- la figure 8 est une vue en coupe suivant la ligne 8-8 de la figure 7 ;
- la figure 9 est une vue en plan représentant une forme de réalisation supplémentaire de l'emballage réfrigérant selon la présente invention ;
- la figure 10 est une vue en coupe suivant la ligne 10-10 de la figure 9 ;
- la figure 11 est une vue en coupe suivant la ligne 11-11 de la figure 9 ;
- la figure 12 est une vue en plan représentant une autre forme de réalisation supplémentaire de l'emballage réfrigérant selon la présente invention ;
- la figure 13 est une vue en coupe suivant la ligne 13-13 de la figure 12 ; et
- la figure 14 est une vue en coupe suivant la ligne 14-14 de la figure 12.

### FORMES PREFEREES DE REALISATION

La présente invention sera maintenant décrite, à titre d'exemples, en se référant aux dessins ci-joints.

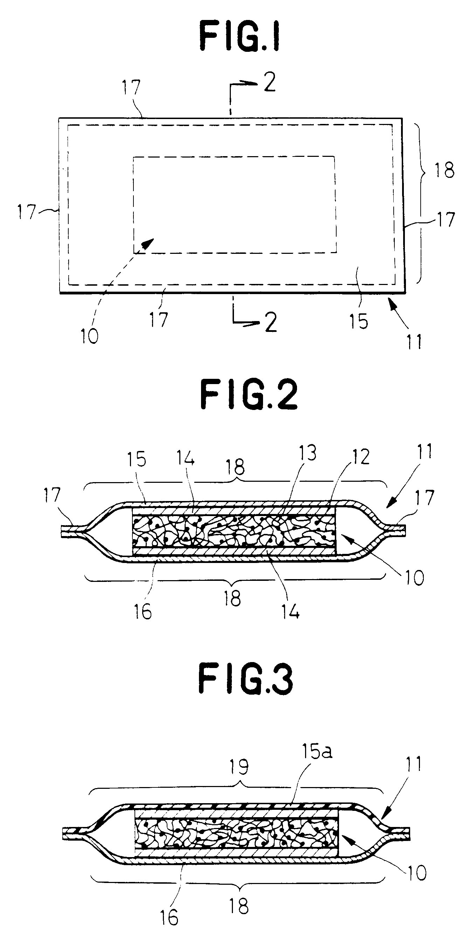

En se référant aux figures 1 et 2, un emballage réfrigérant présentant l'inconvénient auquel on cherche à remédier comprend d'une façon générale un support de réfrigérant 10 se présentant sous la forme d'une feuille ou d'une natte carrée, et une enveloppe 11 enfermant ce support de réfrigérant 10 qui comprend à son tour des particules de polymère 13 absorbant le liquide, ces particules étant dispersées et fixées sur un substrat 12 absorbant le liquide (appelé également ci-après perméable au liquide). Ce support de réfrigérant peut être constitué par une feuille comprenant par exemple des couches de tissu entre lesquelles sont prises en sandwich les particules de polymère absorbant le liquide. Cependant, pour obtenir une absorption uniforme du liquide par le support de réfrigérant, il est préférable d'adapter un support de réfrigérant comprenant un substrat absorbant le liquide, et en particulier un substrat fibreux absorbant le liquide, ce substrat contenant les particules de polymère d'absorption du liquide dispersées et fixées sur celui-ci dans une répartition uniforme.

Lorsqu'on dit que les particules de polymère absorbant le liquide sont dispersées et fixées sur le substrat fibreux, on se réfère à une condition dans laquelle le polymère absorbant le liquide, se présentant de préférence sous la forme de particules sensiblement sphériques, enferme partiellement les fibres élémentaires du substrat fibreux; ces particules du polymère absorbant le liquide étant plus précisément intégrées physiquement aux fibres. Le terme de "répartition uniforme" utilisé ici n'est pas limité à une condition dans laquelle une certaine quantité des particules de polymère absorbant le liquide dispersées et fixées sur le substrat d'absorption du liquide par unité de volume ou par unité de surface de ce dernier, est constante indépendamment des positions considérées, mais concerne également une condition dans laquelle les particules de polymère absorbant le liquide sont dispersées et fixées sur le substrat d'absorption du liquide dans une configuration de points ou de bandes à répétition régulière. D'une façon générale, ce terme désigne une condition dans laquelle les particules de polymère absorbant le liquide sont dispersées et fixées sur le substrat d'absorption du liquide avec une répartition uniforme dans son ensemble.

Les particules de polymère absorbant le liquide utilisables dans la présente invention, doivent être capables d'absorber une quantité d'eau représentant plusieurs dizaines de fois à plusieurs centaines de fois leur propre poids, et peuvent être des polymères en poudre popularisés classiquement sous la forme de différents types d'amidon, de polyacrylate, d'alcool de polyvinyle, et de polyester.

Comme substrat fibreux absorbant le liquide, sur lequel les particules de polymère absorbant le liquide sont dispersées et fixées, on peut utiliser un tissu de papier aéré ou peigné, un tissu non tissé, ou autres. De préférence, des fibres hydrophiles telles que des fibres de pulpe et des fibres de rayonne, des fibres hydrophobes telles que des fibres de polyester et de polypropylène, ou des mélanges ou combinaisons de ces fibres peuvent être utilisés dans une configuration moulée de façon lâche ou prise à chaud, pour obtenir une bonne stabilité de forme du substrat.

La dispersion et la fixation de particules de polymère super absorbantes sur un substrat fibreux, est obtenue plus particulièrement en traitant le substrat fibreux par une solution monomère aqueuse contenant, comme ingrédients principaux, un acrylate ou un mélange d'acrylate/métacrylate de métal alcalin ou d'ammonium, puis en polymérisant ces produits par un déclencheur de polymérisation à radical aqueux. Le substrat fibreux peut être détrempé par la solution monomère aqueuse par étalement ou revêtement de celle-ci.

La pénétration et la diffusion de la solution monomère aqueuse dans le substrat fibreux dépend de différents facteurs tels que la tension superficielle de la solution monomère aqueuse, du poids spécifique et de la densité du substrat fibreux, et de l'angle sous lequel les fibres élémentaires viennent en contact avec la solution aqueuse. Ces facteurs définissent ensemble un état de dispersion et de fixation de la solution monomère aqueuse, et par conséquent du polymère obtenu sur le substrat fibreux. Un facteur supplémentaire important est le taux de rétablissement élastique du substrat fibreux après compression.

Pour obtenir une dispersion et une fixation de la solution monomère aqueuse avec une répartition uniforme, il est préférable d'utiliser un substrat fibreux présentant un poids unitaire de 15 à 50 g/m², et un taux de rétablissement après compression d'au moins 30%.

Avec un substrat fibreux contenant au moins partiellement des fibres hydrophobes destinées à venir en contact avec l'eau sous un angle de 70 à 110°, le taux de rétablissement élastique après compression est amélioré, de sorte que la solution monomère aqueuse et, par conséquent, les particules de polymère, sont maintenues sous une forme sphérique sur le substrat fibreux, avec une répartition uniforme dans tout le substrat fibreux.

Lorsqu'il est mélangé à des fibres fondues à chaud et soumises à une prise à chaud, le substrat fibreux est amélioré en taux de rétablissement élastique après compression, ainsi qu'en solidité et en stabilité de forme. Par exemple, il est possible d'utiliser un substrat fibreux présentant un volume spécifique (en masse) inférieur à 1,5 cm³/g comprenant un tissu mélangé à moins de 45% de fibres de polyester fondu à chaud, puis moulé à chaud et soumis enfin à une prise à chaud.

En variante, cette dispersion et cette fixation des particules de polymère absorbant le liquide sur le substrat fibreux, sont obtenues en mélangeant uniformément les fibres dans la solution monomère aqueuse, puis en polymérisant le mélange de ceux-ci, ou en broyant ou étalant le mélange polymérisé pour le mouler ensuite sous la forme d'une feuille qu'on fait ensuite prendre à chaud.

De préférence, une feuille de diffusion 14 réalisée dans un matériau tel qu'un tissu de papier ou une pulpe broyée, est feuilletée d'un seul tenant sur les surfaces supérieures et/ou inférieure du support de réfrigérant 10 ayant été préparé comme dans les exemples décrits ci-dessus, en comprimant cette feuille de diffusion 14 avec le substrat fibreux 12. Avec le support de réfrigérant 10 ainsi obtenu, on a constaté que le liquide était uniformément absorbé dans toutes les particules de polymère, et que la faculté de retenir le liquide c'est-à-dire, en d'autres termes, la faculté de refroidissement, étaient effectivement améliorées.

L'enveloppe 11 destinée à enfermer le support de réfrigérant 10 décrit ci-dessus, comprend des feuilles perméables au liquide 15, 16 définissant des couches supérieure et inférieure qui sont scellées à chaud le long de leurs pourtours extérieurs 17 pour fermer l'enveloppe 11 de manière étanche.

Cette enveloppe 11 comporte des couches opposées formées par les feuilles perméables au liquide 15, 16 réalisées dans un tissu non tissé, de sorte que les parties de couches perméables au liquide 18 sont formées sur toutes ces couches opposées. Lorsque l'emballage réfrigérant utilisant cette enveloppe 11 est immergé dans un réfrigérant tel que de l'eau, ce réfrigérant est rapidement introduit dans l'enveloppe et absorbé par le support de réfrigérant 10. Les particules de polymère absorbant le liquide 13, qui constituent un élément du support de réfrigérant 10, ont été fixées au substrat fibreux 12 de façon qu'aucune exsudation ou suintement des particules de polymère ne se produisent hors de l'enveloppe 11 avant et après l'introduction du liquide, ce qui permet ainsi d'obtenir l'absorption uniforme voulue du liquide.

L'emballage réfrigérant est ensuite placé dans un appareil de refroidissement tel qu'un réfrigérateur, puis utilisé pour différentes applications de refroidissement après que les particules de polymère absorbant le liquide et retenant celui-ci aient été suffisamment réfrigérées. Les feuilles 15, 16 formant les parties de couches perméables au liquide 18 présentent de préférence non seulement une bonne perméabilité au liquide mais encore une solidité appropriée, et l'on peut utiliser, comme matériau de réalisation de ces feuilles, un tissu non tissé présentant une résistance à la pression d'eau de 200 à 300 mm H₂O ou moins, ou une feuille ou film de matière plastique perforée présentant une grande perméabilité au liquide ou encore, un feuilleté de ces feuilles. De préférence cependant, on utilise un tissu non tissé. Ce tissu non tissé comprend un cordon de liaison, un collage de liaison, une liaison fondue et un tissu non tissé de type sec.

En traitant le tissu non tissé par un surfactant, on améliore notablement la perméabilité au liquide en présence d'une faible pression d'eau. Cependant, un tissu non tissé à grande perméabilité au liquide présente également une faculté élevée de retenir le liquide, ce qui constitue un inconvénient en ce sens que lorsque plusieurs emballages réfrigérants utilisant chacun l'enveloppe réalisée dans ce tissu non tissé, sont empilés dans un appareil de refroidissement, les enveloppes des emballages réfrigérants respectifs collent parfois les uns aux autres lorsque du liquide résiduel retenu dans le tissu non tissé vient à geler, de sorte qu'on ne peut plus séparer les emballages réfrigérants individuels les uns des autres. Par suite, ce résidu de liquide doit être retiré du tissu non tissé par séchage des emballages réfrigérants respectifs avant de les placer dans l'appareil de refroidissement.

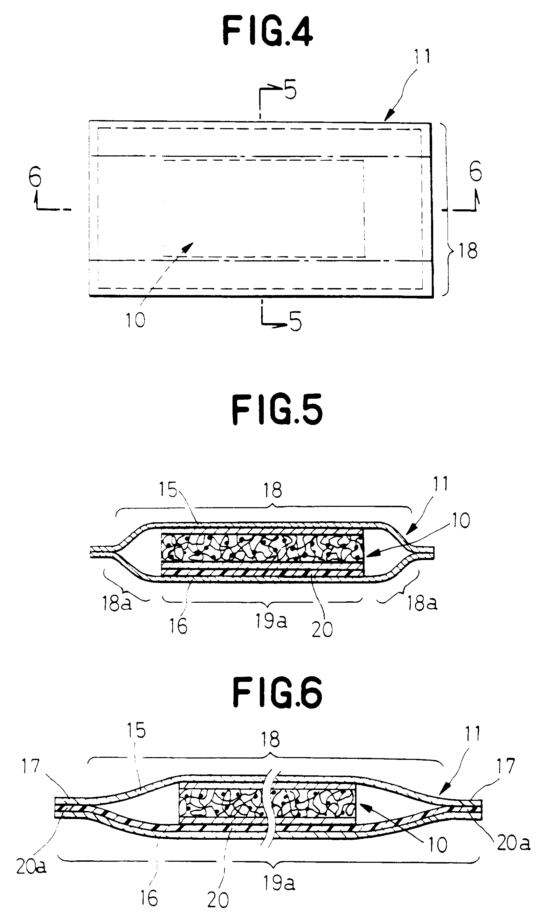

Pour supprimer cette difficulté, une forme de réalisation de l'emballage réfrigérant selon l'invention représentée sur la figure 3, est construite de façon que la couche supérieure de l'enveloppe 11 soit formée par la feuille imperméable au liquide 15a telle que la feuille ou film de matière plastique, tandis que la couche inférieure est formée par la feuille perméable au liquide 16, ce qui définit ainsi respectivement une partie de couche imperméable au liquide 19 et une partie de couche perméable au liquide 18. Les autres caractéristiques sont identiques aux caractéristiques correspondantes de la forme de réalisation représentée sur les figures 1 et 2.

Lorsqu'un certain nombre des emballages réfrigérants construits chacun comme indiqué ci-dessus, sont empilés de manière à amener les parties de couches perméables au liquide 18 en contact avec les parties de couches imperméables au liquide 19, pour refroidir efficacement et simultanément ces emballages, les enveloppes 11 ne collent pas les unes aux autres même si les parties de couches perméables au liquide respectives 18 contiennent une certaine quantité d'eau résiduelle, et si cette eau résiduelle gèle, car la partie de couche imperméable au liquide 19 se trouvant en contact avec la partie de couche perméable au liquide 18 présente une répulsion relativement élevée pour l'eau. Par suite, même si la partie de couche perméable au liquide est réalisée dans un tissu non tissé, le processus indiqué ci-dessus de séchage de ce tissu non tissé n'est pas nécessaire.

Dans la forme de réalisation de l'emballage réfrigérant illustrée sur les figures 4 à 6, dans le même but que dans la forme de réalisation de la figure 3, les couches supérieure et inférieure de l'enveloppe 11 sont formées respectivement par les feuilles perméables au liquide 15, 16, et l'on utilise entre le support de réfrigérant 10 enfermé dans l'enveloppe 11, et la feuille perméable au liquide inférieure 16, une feuille imperméable au liquide 20 telle qu'une feuille ou film de matière plastique présentant sensiblement la même largeur que le support de réfrigérant 10 et s'étendant dans le sens longitudinal de l'enveloppe 11 pour venir longitudinalement en face des extrémités opposées 20a prises en sandwich entre les deux feuilles 15, 16. En scellant à chaud les bords périphériques de feuilles respectives 15, 16 avec les extrémités longitudinalement opposées 20a de la feuille imperméable au liquide 20, la couche supérieure de l'enveloppe définit la couche perméable au liquide 18 et la couche inférieure de l'enveloppe définit la combinaison de la partie de couche perméable au liquide 18a et de la partie de couche imperméable au liquide 19a. Les autres caractéristiques sont identiques aux caractéristiques correspondantes de la forme de réalisation représentées sur les figures 1 et 2.

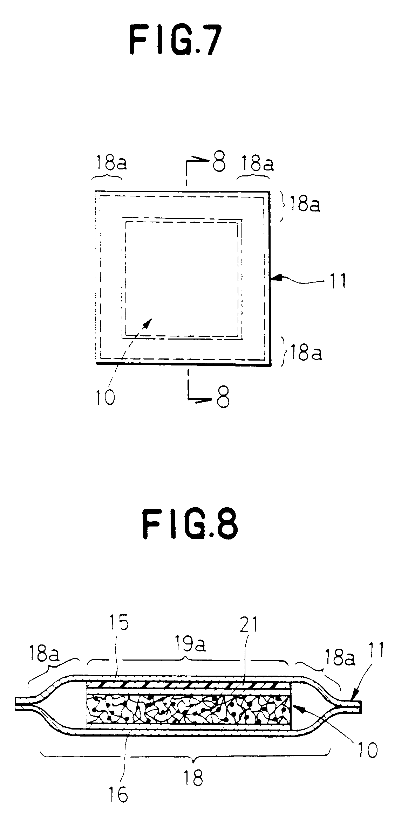

Egalement dans la forme de réalisation de l'emballage réfrigérant représentée sur les figures 7 et 8, essentiellement pour les mêmes raisons que dans la forme de réalisation de la figure 3, les couches supérieure et inférieure de l'enveloppe sont formées respectivement par les feuilles perméables au liquide 15, 16 ; et le côté supérieur au moins du support de réfrigérant 10 enfermé dans l'enveloppe, est recouvert par une feuille imperméable au liquide 21 telle qu'une feuille ou film de matière plastique, de façon que la couche supérieure de l'enveloppe définisse la combinaison de la partie de couche perméable au liquide 18a et de la partie de couche imperméable au liquide 19a, tandis que la couche inférieure de l'enveloppe définit la partie de couche perméable au liquide 18.

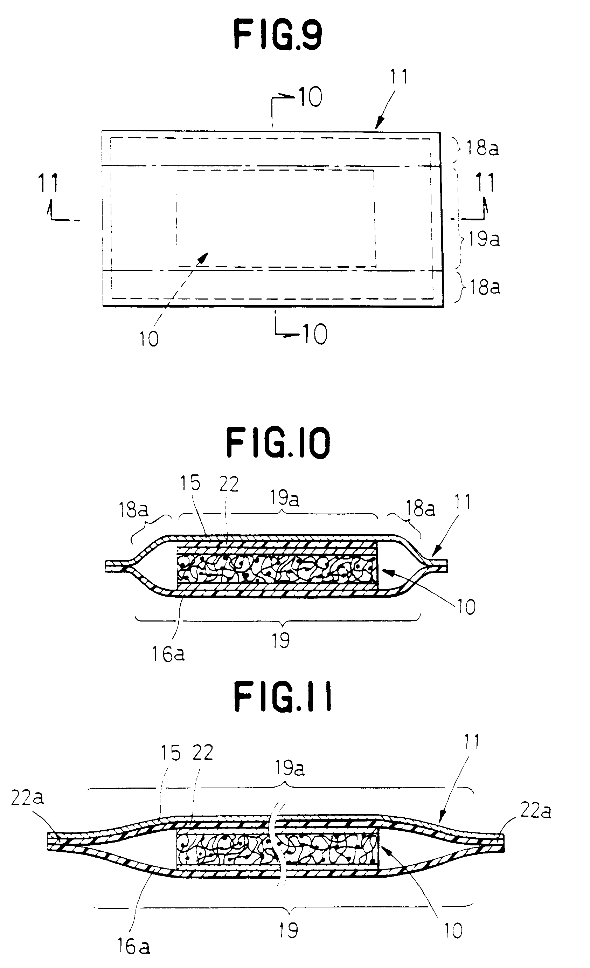

Egalement dans la forme de réalisation de l'emballage réfrigérant représentée sur les figures 9 à 11, pour les mêmes raisons que dans la forme de réalisation de la figure 3, les couches supérieure et inférieure de l'enveloppe 11 sont formées respectivement par la feuille perméable au liquide 15 et par une feuille imperméable au liquide 16a telle qu'une feuille ou film de matière plastique, et l'on utilise entre la feuille supérieure perméable au liquide 15 et le support de réfrigérant 10 enfermé dans l'enveloppe, une feuille imperméable au liquide 22 telle d'une feuille ou film de matière plastique présentant sensiblement la même largeur que celle du support de réfrigérant et s'étendant dans le sens longitudinal de l'enveloppe pour venir longitudinalement en face des extrémités opposées 22a prises en sandwich entre les deux feuilles 15, 16a.

En scellant à chaud les bords périphériques des feuilles respectives 15, 16a avec les extrémités longitudinalement opposées 22a de la feuille imperméable au liquide 22, la feuille supérieure de l'enveloppe définit la combinaison de la partie de couche perméable au liquide 18a et de la partie de couche imperméable au liquide 19a, tandis que la couche inférieure de l'enveloppe définit la partie de couche imperméable au liquide 19. Les autres caractéristiques sont identiques aux caractéristiques correspondantes de la forme de réalisation représentée sur les figures 1 et 2.

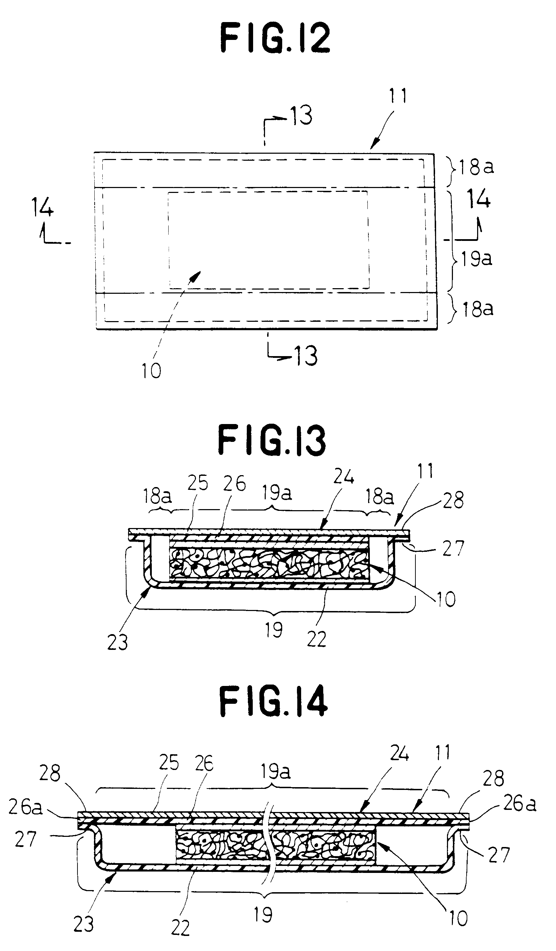

De la même façon, dans la forme de réalisation de l'emballage réfrigérant illustrée sur les figures 12 à 14, pour les mêmes raisons que dans la forme de réalisation de la figure 3, l'enveloppe 11 consiste en un corps en forme de boîte ouvert vers le haut 23 formé par des feuilles de matière plastique, et un capot 24 comprenant à son tour un tissu fibreux non tissé 25 et une feuille imperméable au liquide 26 telle qu'une feuille ou film de matière plastique collé à la surface intérieure du tissu non tissé 25 sauf sur les surfaces des côtés opposés transversalement qui présentent chacune une largeur appropriée. Le corps en forme de boîte 23 de l'enveloppe 11 contient le support de réfrigérant 10 et supporte le capot 24 sur les rebords 27 le long des extrémités longitudinalement opposées du corps en forme de boîte 23. Ainsi, les extrémités longitudinalement opposées 26a de la feuille imperméable au liquide 26 sont prises en sandwich entre les rebords 27 et les extrémités longitudinalement opposées 28 du capot 24. Le corps en forme de boîte 23 et le capot 25 sont scellés à chaud le long de leurs bords périphériques, avec les extrémités longitudinalement opposées 26a de la feuille imperméable au liquide 26, de manière à fermer l'enveloppe 11 de manière étanche. De cette façon, la partie de couche perméable au liquide 18a est définie par les bords latéraux transversalement opposés du capot 24, et les parties de couche imperméable au liquide 19a, 19 sont définies par le reste de l'enveloppe 11. Les autres caractéristiques sont identiques aux caractéristiques correspondantes de la forme de réalisation représentée sur les figures 1 et 2.

Comme cela apparaît clairement sur les emballages réfrigérants représentés par les figures 6 à 14, les feuilles imperméables au liquide 20, 21, 22, 26 peuvent être placées, au moins au centre, sur la couche supérieure ou inférieure de chaque emballage réfrigérant empilé sur les autres, ce qui permet d'obtenir l'objet voulu.

L'expression "partie de couche imperméable au liquide" utilisée ici, ne doit pas nécessairement impliquer, au sens strict, "imperméabilité parfaite au liquide", mais concerne différentes parties de couches présentant des caractéristiques telles qu'une résistance relativement élevée à la pression d'eau, un passage libre réduit du liquide à travers ces parties de couches, ou une possibilité réduite au minimum de comporter des résidus liquides dans ces parties de couches.

Par exemple, lorsqu'une partie de couche de tissu non tissé porte partiellement sur sa surface intérieure une feuille ou film de matière plastique comme indiqué dans les formes de réalisation ci-dessus, cette partie de couche devient pratiquement imperméable au liquide lorsque le tissu non tissé vient en contact étroit avec la feuille ou film de matière plastique sous une pression d'eau introduite dans l'enveloppe. En particulier, lorsque cette partie de couche est réalisée dans un tissu non tissé hydrophobe, le résidu de liquide retenu dans la partie de couche portant la feuille ou le film de matière plastique, est sensiblement inférieur à celui qui reste dans les autres parties de la couche. Par suite, si un certain nombre d'emballages réfrigérants sont empilés de façon que ces parties de couches soient en contact avec un autre emballage réfrigérant pendant qu'on effectue le refroidissement, le problème souvent rencontré dans l'art antérieur, selon lequel les emballages réfrigérants individuels se collent les uns aux autres de manière inséparable, est effectivement supprimé. Si cela est nécessaire, la feuille ou film de matière plastique peut être collée au tissu non tissé sur toute sa surface ou sur un dessin convenable.

Comme on le comprendra d'après les formes de réalisation illustrées ici, le terme d'"enveloppe" utilisé ici désigne une enveloppe en forme de sac, en forme de boîte ou analogue, réalisée dans un matériau flexible ou rigide et comportant au moins une paire de couches opposées l'une à l'autre, ces couches étant sensiblement plates (ou pouvant être légèrement courbes dans la mesure où elles peuvent se déformer pour prendre une position plate) de façon qu'on puisse empiler verticalement ou horizontalement un certain nombre d'emballages réfrigérants.

De préférence, les volumes du support de réfrigérant 10 et de l'enveloppe 11 sont dimensionnés de façon que l'enveloppe puisse s'adapter effectivement au support de réfrigérant même après que ce dernier ait absorbé une quantité convenable de liquide et que son volume ait augmenté en conséquence. Sinon, l'emballage réfrigérant pourrait parfois se déformer malencontreusement du fait de cette augmentation de volume du support de réfrigérant, bien que cela dépende de la force de ce dernier.

On comprendra que les feuilles imperméables au liquide 20, 21, 22, 26 peuvent être remplacées par une feuille d'aluminium si on le désire.

## Revendications

1. Emballage réfrigérant destiné à être congelé dans un appareil de refroidissement et comportant en combinaison,
- d'une part
un support de réfrigérant (10) se présentant sous la forme d'une feuille ou d'une natte qui est composé de :
- a) un substrat absorbant le liquide (12) réalisé sous la forme d'une masse de matériau fibreux et,
- b) des particules de polymère (13) absorbant le liquide se présentant sous la forme de particules sensiblement sphériques dispersées et fixées sur le substrat (12) en enfermant partiellement les fibres de celui-ci, ces particules étant capables d'absorber une quantité d'eau représentant plusieurs dizaines de fois à plusieurs centaines de fois leur propre poids, et
- d'autre part
une enveloppe (11) en forme de sac de boîte ou analogue constituée à sa partie externe par une paire de couches (15, 15a ; 16, 16a ; 23, 24) opposées l'une à l'autre et scellées au niveau de leurs pourtours extérieurs (17, 27) pour fermer l'enveloppe (11) en enfermant le support réfrigérant (10),
emballage réfrigérant caractérisé en ce qu'au moins l'une des couches (15, 16, 24) d'une paire est réalisée en un matériau perméable aux liquides de manière à définir une partie de couches (18, 18a) perméable aux liquides permettant l'introduction de liquides à l'intérieur de l'enveloppe (11) et en ce que l'enveloppe (11) comporte une partie de couches (19, 19a) imperméable aux liquides, les parties de couches perméables aux liquides (18, 18a) et imperméables aux liquides (19, 19a) respectives d'enveloppes (11) d'emballages réfrigérants empilés dans un appareil de refroidissement coopérant de façon à éviter tout collage de ces emballages.

2. Emballage réfrigérant selon la revendication 1, caractérisé en ce que l'une des couches (15a, 16a, 23) d'une paire est réalisée en un matériau imperméable aux liquides et définit au moins partiellement la partie de couches (19) imperméable aux liquides.

3. Emballage réfrigérant selon l'une quelconque des revendications 1 et 2, caractérisé en ce que l'enveloppe (11) comporte au moins une couche (15, 16, 24) réalisée en un matériau perméable aux liquides et coopérant au moins à sa partie centrale avec une feuille imperméable aux liquides (20, 21, 22, 26) interposée entre cette couche (15, 16, 24) et le support de réfrigérant (10), et définissant au moins partiellement la partie de couches (19a) imperméable aux liquides.

4. Emballage réfrigérant selon la revendication 3, caractérisé en ce que la portion périphérique de la couche (15, 16, 24) indépendante de la feuille (20, 21, 22, 26) imperméable aux liquides définit au moins partiellement la partie de couches (18a) perméable aux liquides.

5. Emballage réfrigérant selon l'une quelconque des revendications 1 à 4, caractérisé en ce que le matériau perméable aux liquides est un tissu fibreux non tissé.

6. Emballage réfrigérant selon l'une quelconque des revendications 1 à 5, caractérisé en ce que le matériau imperméable aux liquides est choisi dans le groupe formé par les matières plastiques et les métaux tels que l'aluminium.

## Claims

1. A refrigerated parcel intended to be frozen in a cooling device and comprising in combination,
in one part
a refrigerating support (10) provided in the form of a foil or a mat which is comprised of:
a) a liquid-absorbing substrate (12) produced in the form of a mass of fibrous material and,
b) liquid absorbing polymer particles (13) provided in the form of particles which are approximately spherical in shape, dispersed and fixed on the substrate (12) partially enclosing the fibres of this substrate, these particles being capable of absorbing a quantity of water representing several dozen times to several hundred times their own weight, and
in another part
a jacket (11) in the form of an enclosing bag or analogous, composed in its external part of a pair of opposing layers (15, 15a ; 16, 16a ; 23, 24) sealed at the region of their outer periphery (17, 27) to close the jacket (11) thus enclosing the refrigerating support (10),
characterised in that at least one of the layers (15, 16, 24) of a pair is produced of a material which is permeable to liquids so as to define a region of layers (18, 18a) which is permeable to liquids allowing the introduction of liquids to the inside of the jacket (11), and in that the jacket (11) comprises a region of layers (19, 19a) which is impermeable to liquids, the respective regions of layers which are permeable to liquids (18, 18a) and impermeable to liquids (19, 19a) of jackets (11) of refrigerated parcels stacked in a cooling device cooperating so as to avoid any adherence of these parcels to each other.

2. A refrigerated parcel according to claim 1, characterised in that one of the layers (15a, 16a, 23) of a pair is produced of a material which is impermeable to liquids and defines at least partially the region of layers (19) which is impermeable to liquids.

3. A refrigerated parcel according either of claims 1 and 2, characterised in that the jacket (11) comprises at least one layer (15, 16, 24) produced in a material which is permeable to liquids and cooperates at least in its central part with a foil which is impermeable to liquids (20, 21, 22, 26) interposed between this layer (15, 16, 24) and the refrigerating support (10), and defining at least partially the region of layers (19a) which is impermeable to liquids.

4. A refrigerated parcel according to claim 3, characterised in that the peripheral portion of the layer (15, 16, 24) independent of the foil (20, 21, 22, 26) impermeable to liquids defines at least partially the region of layers (18a) which is permeable to liquids.

5. A refrigerated parcel according to any one of claims 1 to 4, characterised in that the material which is permeable to liquids is a nonwoven fibrous fabric.

6. A refrigerated parcel according to any one of claims 1 to 5, characterised in that the material which is impermeable to liquids is chosen from the group made up of plastics and metals such as aluminium.

## Patentansprüche

1. Kühlpackung zum Vereisen in einem Gefrierapparat mit in Kombination
- einerseits einem Kühlmittelträger (10) in Form einer Folie oder Matte, bestehend aus:
a) einem Flüssigkeit aufsaugenden Substrat (12) in Form einer Masse faserigen Materials und
b) die Flüssigkeit absorbierenden, im wesentlichen kugelförmigen Polymerpartikeln (13), die auf dem Substrat (12) verteilt und festgelegt sind, indem sie dessen Fasern teilweise einschließen, und die in der Lage sind, eine Menge Wasser zu absorbieren, welche das mehrmals Zehnfache bis mehrmals Hundertfache des eigenen Gewichts darstellt, und
- andererseits einer Hülle (11) in Form eines Beutels, eines Kastens o. dgl., die in ihrem äußeren Bereich durch ein Paar gegenüberliegender Lagen (15, 15a; 16, 16a; 23, 24) gebildet ist, welche an ihren äußeren Rändern (17, 27) versiegelt sind, um die Hülle (11) zu schließen und damit den Kühlmittelträger (10) einzuschließen,
**dadurch gekennzeichnet,** daß wenigstens eine der beiden Lagen (15, 16, 24) aus einem für Flüssigkeiten durchlässigen Material besteht, derart, daß ein für Flüssigkeiten durchlässiger Lagenbereich (18, 18a) gebildet ist, welcher das Einführen von Flüssigkeiten in das Innere der Hülle (11) zuläßt, und die Hülle (11) einen für Flüssigkeiten undurchlässigen Lagenbereich (19, 19a) aufweist, wobei die für Flüssigkeiten durchlässigen Lagenbereiche (18, 18a) und die für Flüssigkeiten undurchlässigen Lagenbereiche (19, 19a) der Hüllen (11) von in einem Gefrierapparat gestapelten Kühlpackungen so zusammenwirken, daß kein Zusammenfrieren der Packungen eintritt.

2. Kühlpackung nach Anspruch 1, **dadurch gekennzeichnet,** daß eine der beiden Lagen (15a, 16a, 23) aus einem für Flüssigkeiten undurchlässigen Material besteht und wenigstens teilweise den für Flüssigkeiten undurchlässigen Lagenbereich (19) bildet.

3. Kühlpackung nach Anspruch 1 oder 2, **dadurch gekennzeichnet,** daß die Hülle (11) wenigstens eine Lage (15, 16, 24) aufweist, die aus einem für Flüssigkeiten durchlässigen Material besteht und wenigstens in ihrem mittleren Bereich mit einer für Flüssigkeiten undurchlässigen Folie (20, 21, 22, 26) zusammenwirkt, die zwischen dieser Lage (15, 16, 24) und dem Kühlmittelträger (10) eingesetzt ist und wenigstens teilweise den für Flüssigkeiten undurchlässigen Lagenbereich (19a) bestimmt.

4. Kühlpackung nach Anspruch 3, **dadurch gekennzeichnet,** daß der Umfangsbereich der Lage (15, 16, 24), unabhängig von der für Flüssigkeiten undurchlässigen Folie (20, 21, 22, 26), wenigstens teilweise den für Flüssigkeiten durchlässigen Lagenbereich (18a) bildet.

5. Kühlpackung nach einem der Ansprüche 1 bis 4, **dadurch gekennzeichnet,** daß das für Flüssigkeiten durchlässige Material ein faseriger Vliesstoff ist.

6. Kühlpackung nach einem der Ansprüche 1 bis 5, **dadurch gekennzeichnet,** daß das für Flüssigkeiten undurchlässige Material Kunststoff oder Metall, z. B. Aluminium, ist.
